# FASCICULE DE BREVET EUROPEEN

(11) **EP 3 870 200 B1**
(45) Date de publication et mention de la délivrance du brevet: **08.10.2025**
(21) Numéro de dépôt: 19787301.1
(22) Date de dépôt: 22.10.2019
(51) Int. Cl.: A61K 36/23, A61P 3/04, A61K 36/738

(54) **COMPOSITION POUR TRAITER LE SURPOIDS ET/OU L'OBESITE**
ZUSAMMENSETZUNG ZUR BEHANDLUNG VON ÜBERGEWICHT UND/ODER ADIPOSITAS
COMPOSITION FOR TREATING OVERWEIGHT AND/OR OBESITY

(30) Priorité: 22.10.2018 FR 1859711
(43) Date de publication de la demande: 01.09.2021
(73) Titulaire: Nexira, 76000 Rouen (FR)
(72) Inventeur: GUILLEMET, Damien, 76000 Rouen (FR)
(74) Mandataire: Cabinet Netter
(86) Numéro de dépôt international: PCT/EP2019/078746
(87) Numéro de publication internationale: WO 2020/083923

(56) Documents cités:
- EP-A1- 1 616 873
- EP-A2- 1 502 598
- CN-A- 103 564 487
- CN-A- 104 171 813
- JP-A- 2006 016 312
- JP-A- 2017 088 589
- JP-B2- 3 790 767
- JP-B2- 3 790 767
- JP-B2- 5 830 316
- JP-B2- 5 830 316
- RO-A2- 126 833
- RO-B1- 126 833
- DE LA GARZA ANA LAURA ET AL: "Helichrysum and Grapefruit Extracts Boost Weight Loss in Overweight Rats Reducing Inflammation", JOURNAL OF MEDICINAL FOOD, vol. 18, no. 8, 1 August 2015 (2015-08-01), US, pages 890 - 898, XP055945924, ISSN: 1096-620X, Retrieved from the Internet <URL:https://www.liebertpub.com/doi/pdf/10.1089/jmf.2014.0088?casa_token=ihg7vbqD0gkAAAAA:L0K7f3uU5HrVWVLS90esjXKKDMfWQ2Benn5Hy2BYzBp81sFvFvclSkPcBntLs1bUZH5AKPUbp4Y> DOI: 10.1089/jmf.2014.0088
- GUILLEMET DAMIEN ET AL: "Screening for anti-adipogenic, pro-lipolytic and thermogenic plant extracts by models associating intestinal epithelial cells with human adipose cells", EUROPEAN JOURNAL OF NUTRITION, vol. 61, no. 4, 1 June 2022 (2022-06-01), DE, pages 2201 - 2215, XP055945927, ISSN: 1436-6207, Retrieved from the Internet <URL:https://link.springer.com/content/pdf/10.1007/s00394-021-02794-8.pdf> DOI: 10.1007/s00394-021-02794-8
- DE LA GARZA ANA LAURA ET AL: "Helichrysum and Grapefruit Extracts Boost Weight Loss in Overweight Rats Reducing Inflammation", vol. 18, no. 8, 1 August 2015 (2015-08-01), US, pages 890 - 898, XP055945924, ISSN: 1096-620X, Retrieved from the Internet <URL:https://www.liebertpub.com/doi/pdf/10.1089/jmf.2014.0088?casa_token=ihg7vbqD0gkAAAAA:L0K7f3uU5HrVWVLS90esjXKKDMfWQ2Benn5Hy2BYzBp81sFvFvclSkPcBntLs1bUZH5AKPUbp4Y> DOI: 10.1089/jmf.2014.0088
- GUILLEMET DAMIEN ET AL: "Screening for anti-adipogenic, pro-lipolytic and thermogenic plant extracts by models associating intestinal epithelial cells with human adipose cells", vol. 61, no. 4, 1 June 2022 (2022-06-01), DE, pages 2201 - 2215, XP055945927, ISSN: 1436-6207, Retrieved from the Internet <URL:https://link.springer.com/content/pdf/10.1007/s00394-021-02794-8.pdf> DOI: 10.1007/s00394-021-02794-8
- DANIEL ANTUNES VIEGAS ET AL: "Helichrysum italicum: From traditional use to scientific data", JOURNAL OF ETHNOPHARMACOLOGY, vol. 151, no. 1, 1 January 2014 (2014-01-01), IE, pages 54 - 65, XP055320080, ISSN: 0378-8741, DOI: 10.1016/j.jep.2013.11.005
- KESHAV RAJ PAUDEL ET AL: "Phytochemical Profile and Biological Activity of Nelumbo nucifera", EVIDENCE-BASED COMPLEMENTARY AND ALTERNATIVE MEDICINE, vol. 2015, 1 January 2015 (2015-01-01), US, pages 1 - 16, XP055595733, ISSN: 1741-427X, DOI: 10.1155/2015/789124
- NOBUTOMO IKARASHI ET AL: "Anti-Obesity and Anti-Diabetic Effects of Acacia Polyphenol in Obese Diabetic KKAy Mice Fed High-Fat Diet", EVIDENCE-BASED COMPLEMENTARY AND ALTERNATIVE MEDICINE, vol. 2011, 1 January 2011 (2011-01-01), US, pages 1 - 10, XP055641192, ISSN: 1741-427X, DOI: 10.1093/ecam/nep241
- K R SRIDHAR ET AL: "Lotus ? A potential nutraceutical source", JOURNAL OF AGRICULTURAL TECHNOLOGY JOURNAL OF AGRICULTURAL TECHNOLOGY, 1 January 2007 (2007-01-01), pages 143 - 155, XP055595739, Retrieved from the Internet <URL:http://ijat-aatsea.com/pdf/JUN_V3_07/13-IJAT2007_07-R.pdf>
- ANONYMOUS: "< Le poivre des Alpes >, un mélange de d'herbes et de plantes des montagnes fabriqué, en Isère", 5 October 2017 (2017-10-05), XP093033075, Retrieved from the Internet <URL:https://france3-regions.francetvinfo.fr/auvergne-rhone-alpes/isere/poivre-alpes-melange-herbes-plantes-montagnes-fabrique-isere-1341251.html> [retrieved on 20230320]
- ANONYMOUS: "Isabelle Légé, épicière cueilleuse à Grenoble | Elle crapahute sans sourciller de pente en pente son seau à la main. À l'intérieur des graines de carottes sauvages, du genévrier ou encore du... | By France 3 Auvergne | Facebook", 10 October 2017 (2017-10-10), XP093033087, Retrieved from the Internet <URL:https://m.facebook.com/france3auvergne/videos/1604181722985873/?locale=ms_MY> [retrieved on 20230320]
- ANONYMOUS: "Acacia Catechu - Uses & Benefits | Always Ayurveda", 14 August 2018 (2018-08-14), XP055640928, Retrieved from the Internet <URL:https://web.archive.org/web/20180814053859/http://www.alwaysayurveda.com/acacia-catechu/> [retrieved on 20191111]
- SWAYAM PRAKASH SRIVASTAVA ET AL: "hard wood: potential anti-diabetic cum anti-dyslipidemic", MEDICINAL CHEMISTRY RESEARCH, BIRKHÄUSER-VERLAG, BOSTON, vol. 20, no. 9, 12 December 2010 (2010-12-12), pages 1732 - 1739, XP019971292, ISSN: 1554-8120, DOI: 10.1007/S00044-010-9479-Y
- JOÃO CARLOS DA SILVA DIAS: "Nutritional and Health Benefits of Carrots and Their Seed Extracts", FOOD AND NUTRITION SCIENCES, vol. 05, no. 22, 1 January 2014 (2014-01-01), pages 2147 - 2156, XP055641324, ISSN: 2157-944X, DOI: 10.4236/fns.2014.522227
- KAMLESH SINGH ET AL: "HYPOLIPIDEMIC ACTIVITY OF ETHANOLIC EXTRACT OF DAUCUS CAROTA SEEDS IN NORMAL RATS", INTERNATIONAL JOURNAL OF BIOMEDICAL AND ADVANCE RESEARCH, vol. 1, no. 3, 1 January 2010 (2010-01-01), pages 1 - 3, XP055641325, DOI: 10.7439/ijbar.v1i3.4
- ANONYMOUS: "HELICHRYSUM ITALICUM Properties", 18 January 2018 (2018-01-18), XP055595695, Retrieved from the Internet <URL:https://web.archive.org/web/20180118123256/https://helichrysum-italicum.com/properties-3-w.asp> [retrieved on 20190612]
- NINOMIYA ET AL: "Potent anti-obese principle from Rosa canina: Structural requirements and mode of action of trans-tiliroside", BIOORGANIC & MEDICINAL CHEMISTRY LETTERS, PERGAMON, AMSTERDAM, NL, vol. 17, no. 11, 10 May 2007 (2007-05-10), pages 3059 - 3064, XP022068393, ISSN: 0960-894X, DOI: 10.1016/J.BMCL.2007.03.051
- DATABASE GNPD [online] MINTEL; 11 January 2008 (2008-01-11), ANONYMOUS: "Purifying Herbal Tea", XP055672199, retrieved from www.gnpd.com Database accession no. 841672
- DATABASE GNPD [online] MINTEL; 9 November 2017 (2017-11-09), ANONYMOUS: "Orange, Carrot and Pineapple Infusion", XP055672201, retrieved from www.gnpd.com Database accession no. 5234929
- AKIFUMI NAGATOMO ET AL: "Daily intake of rosehip extract decreases abdominal visceral fat in preobese subjects: a randomized, double-blind, placebo-controlled clinical trial", DIABETES, METABOLIC SYNDROME AND OBESITY: TARGETS AND THERAPY, 1 March 2015 (2015-03-01), pages 147, XP055672205, DOI: 10.2147/DMSO.S78623

## Description

La présente invention se rapporte à une composition de complément alimentaire comprenant au moins un extrait comestible de plante. L'invention se rapporte également à l'utilisation de cette composition pour traiter le surpoids et/ou l'obésité.

La gestion des graisses dans le corps humain est régulée par le tissu adipeux, et plus particulièrement par les adipocytes qui sont les cellules qui composent ce tissu. Cette régulation implique une pluralité de mécanismes cellulaires, parmi lesquels on trouve des mécanismes pour le stockage de graisses, et des mécanismes pour la libération, la décomposition et/ou la transformation de graisses.

En particulier, il existe la lipogenèse, la lipolyse, l'adipogénèse et la thermogenèse.

La lipogenèse comprend essentiellement la transformation d'acides gras libres en triglycérides pour le stockage dans les adipocytes. La lipolyse comprend essentiellement l'hydrolyse des triglycérides stockés pour libérer et évacuer (c'est à dire relibérer dans la circulation sanguine) des graisses des adipocytes. L'adipogenèse est le mécanisme cellulaire dans lequel des cellules appelées préadipocytes se différencient en adipocytes pour augmenter le stockage de graisses dans le corps.

Dans la thermogenèse, les adipocytes transforment des graisses directement en chaleur. Plus généralement, la thermogenèse est la production de chaleur du corps humain par augmentation du métabolisme cellulaire. La thermogenèse intervient notamment lorsque la température du corps est inférieure au point de consigne, qui est d'environ 37°C. La thermogenèse se déroule, au moins en partie, dans une certaine classe d'adipocytes appelés adipocytes bruns. Il s'agit en pratique d'une transformation des graisses en chaleur, cf. Fenzl A., Kiefer FW., Brown adipose tissue and thermogenesis, Horm Mol Biol Clin Investig., 2014, 19(1):25-37.

D'une manière générale, le métabolisme des adipocytes a un effet majeur sur le métabolisme global du corps humain. Les personnes ayant un excès de tissu adipeux présentent ainsi généralement des problèmes de santé. Plus globalement, l'excès de masse graisseuse entraîne des inconvénients pour la santé. Cet excès est appelé surpoids et peut aller jusqu'à l'obésité, cf. Brinderjit Kaila and Maitreyi Raman, MD FRCPC Obesity: A review of pathogenesis and management strategies, Can J Gastroenterol, 2008, 22(1):61-68 et Lean M. et al., Making progress on the global crisis of obesity and weight management, BMJ, 2018, 361. Aujourd'hui, environ 30% de la population est atteinte de surpoids et d'obésité.

L'état de la technique décrit des plantes ayant un effet sur le tissu adipeux.

En particulier, le café et le thé ont des effets sur le tissu adipeux. Les effets sont principalement liés à la présence de caféine dans ces plantes. La caféine est notamment impliquée dans l'activation de la lipolyse et la stimulation générale du métabolisme des adipocytes, cf. M.-H. Pan, Y.-C. Tung, G. Yang, S. Li, C.-T. Ho, Molecular mechanisms of the anti-obesity effect of bioactive compounds in tea and coffee, 2016, Food Funct., 2016, Nov 9;7(11):4481-4491.

Le café vert présente en outre l'avantage de comporter des polyphénols, ce qui semble favoriser au moins en partie la thermogenèse. En conséquence, le café vert trouve une application dans le traitement de personnes atteintes de surpoids ou d'obésité.

Toutefois, le dosage journalier relatif à la caféine est difficilement contrôlable. Ceci est notamment dû à la présence de cette molécule dans de nombreux aliments. Un suivi précis est difficile, voire impossible. De plus, au-delà de son effet sur le tissu adipeux, la caféine a des effets sur d'autres processus métaboliques chez l'humain. De façon générale, la caféine est connue pour être un stimulant et un psychostimulant. Elle a un effet sur le système cardiovasculaire qui peut entraîner une accélération néfaste du rythme cardiaque. Dans certains cas, la caféine a un effet diurétique. Elle est en outre antagoniste des récepteurs du neurotransmetteur adénosine dans le système nerveux central. Ainsi, la caféine peut générer des dépendances physique et psychologique chez certaines personnes.

Tout ceci fait que le café ou thé ne sont pas entièrement satisfaisants pour le traitement de personnes atteintes de surpoids ou d'obésité. De plus, la caféine stimule la lipolyse, c'est-à-dire le processus d'hydrolyse de triglycérides stockés dans les adipocytes pour libérer et évacuer des graisses. Dans le corps, les graisses sont donc relâchées par les adipocytes dans la circulation sanguine générale. Ceci peut notamment entraîner des problèmes cardiovasculaires ou des dysfonctionnements hépatiques.

D'autres plantes semblent avoir un effet sur le métabolisme du tissu adipeux.

Le document EP 3 096 767 divulgue une composition comprenant de l'okra. La composition réduit l'absorption des graisses par le corps. Pour cela, la composition doit être prise lors des repas, ou au moins être prise proche des repas (15 à 30 minutes avant, ou 30 à 45 minutes après le repas).
**Le document *"***Potent anti-obese principle from Rosa canina: Structural requirements and mode of action of trans-tiliroside", Kiyofumi Ninomiya et al., Bioorganic & Medicinal Chemistry Letters 17 (2007) 3059-3064 **divulgue des effets anti-obésité de *Rosa Canina.***

Il existe un besoin constant d'identifier de nouveaux composés ayant un effet sur le tissu adipeux.

Tout particulièrement, il existe un besoin d'identifier des composés capables d'activer ou de stimuler la thermogenèse. En effet, la thermogenèse transforme les graisses en chaleur. Elle ne présente donc notamment pas des inconvénients liés à la lipolyse.

La présente invention vient améliorer la situation La composition de i'invention est définie dans les revendications annexées.

À cet effet, la présente description divulgue une composition comprenant un extrait comestible d'au moins une des plantes choisies parmi le groupe constitué de *Nelumbo nucifera, Mangifera indica, Acacia catechu, Commiphora mukul, Daucus carota, Cinnamomum verum, Rosa canina,* et *Helichrysum italicum.*

Dans la présente description, la composition peut comprendre un extrait comestible d'au moins deux desdites plantes.

Dans la description, la composition peut comprendre un extrait comestible d'au moins trois desdites plantes ou un extrait comestible de quatre desdites plantes.

Dans la description la composition peut comprendre un extrait comestible de deux desdites plantes formant ainsi une combinaison d'extraits de plantes, ladite combinaison étant choisie parmi :
- une combinaison de *Acacia catechu* et *Commiphora mukul ;*
- une combinaison de *Acacia catechu* et *Helichrysum italicum ;*
- une combinaison de *Nelumbo nucifera* et *Helichrysum italicum ;*
- une combinaison de *Daucus carota* et *Rosa canina ;*
- une combinaison de *Acacia catechu* et *Rosa canina ;* et
- une combinaison de *Daucus carota* et *Cinnamomum verum.*

Dans la description, la ou les extraits comestibles proviennent respectivement :
- de la fleur de *Nelumbo nucifera ;*
- de la peau du fruit de *Mangifera indica ;*
- de l'écorce *d' Acacia catechu ;*
- de l'exsudat de *Commiphora mukul ;*
- de la graine de *Daucus carota ;*
- de la feuille de *Cinnamomum verum ;*
- du fruit de *Rosa canina ;*
- de la partie aérienne de *Helichrysum italicum.*

La composition de l'invention est pour l'utilisation dans le traitement et/ou la prévention d'une affection choisie parmi, l'obésité et une maladie métabolique chez un humain ou un animal.

La ou les extraits comestibles peuvent être sous forme pulvérulente ou granulaire dont le diamètre granulométrique d90 des particules est inférieur ou égale à 180 µm.

La composition peut comprendre 0,001% à 99% en poids, de préférence 10% à 80% en poids, du ou des extraits comestibles.

La composition peut être sous une forme choisie parmi un comprimé, un cachet, une capsule, un granule, une gélule, une dragée, une gomme à mâcher, une pâte, une boisson, un sirop et une poudre.

L'invention peut être sous forme d' un produit alimentaire, ou complément alimentaire, ou complément diététique, ou substitut de repas, ou boisson, ou supplément de boisson, ou produit pharmaceutique, comprenant la composition décrite ci-dessus.

Dans un mode de réalisation, le produit pharmaceutique comprend en outre un ou plusieurs supports et/ou excipients.

L'invention vise également un procédé non-thérapeutique pour contrôler le poids corporel d'un sujet, ledit procédé comprenant l'administration d'une quantité efficace d'une composition de l'invention.

D'autres avantages et caractéristiques de l'invention apparaîtront à la lecture de la description détaillée ci-après et sur les dessins annexés sur lesquels :
- La figure 1 montre un schéma d'un système de test de perméabilité ;
- la figure 2 montre un tableau comprenant des extraits de plante et leurs effets respectifs sur une partie du métabolisme adipeux ; et
- la figure 3 montre un tableau comprenant des combinaisons d'extraits de plante et leurs effets respectifs sur une partie du métabolisme adipeux.

Les figures, tableaux et la description ci-après contiennent, pour l'essentiel, des éléments de caractère certain. Les figures et tableaux font partie intégrante de la description, et pourront donc non seulement servir à mieux faire comprendre la présente invention, mais aussi contribuer à sa définition, le cas échéant.

Dans la présente description on entend par "principe actif" une molécule ou une combinaison de molécules ayant un effet bénéfique sur le traitement de surpoids ou de l'obésité. En particulier, il s'agit d'une molécule ou d'une combinaison de molécules ayant un effet sur les adipocytes de sorte à engager ou provoquer :
- la thermogenèse,
- la lipolyse,
- l'inhibition de l'adipogenèse, et/ou
- un effet anti-accumulation lipidique.

Dans la présente description on entend par des sujets ou personnes atteintes de "surpoids" des sujets ou personnes dont l'indice de masse corporelle (IMC, ou BMI pour *Body Mass Index* en anglais) est compris entre 25 et 30.

Dans la présente description on entend par des sujets ou personnes atteintes "d'obésité" des sujets ou personnes dont l'indice de masse corporelle (IMC, ou BMI pour *Body Mass Index* en anglais) est supérieur ou égal à 30.

Le surpoids et l'obésité sont parfois qualifiés "d'affection" pour désigner leur nature correspondante à un trouble de santé.

Dans la présente description le terme "partie comestible" de plante désigne une partie de plantes pouvant être ingérée par un humain *per os,* c'est-à-dire par voie orale ou plus généralement par voie gastro-intestinale. Une telle "partie comestible" peut en outre être ingérée par un animal. La composition de l'invention comprend des extraits de parties comestibles de plantes.

De manière générale, une plante présente deux parties principales : une partie souterraine qui comprend en particulier la racine (ou le tubercule ou le rhizome), et une partie aérienne qui est en particulier composée de la tige, des feuilles, des bourgeons, des fruits et/ou des fleurs.

Une partie comestible d'une plante peut se présenter sous forme solide ou liquide. Il peut donc notamment s'agir de feuilles, d'une racine, d'une tige, d'un fruit, de fleurs ou encore de l'exsudat ou de sève d'une plante, transformé(e) (s) ou non. Il peut s'agir de tout ou seulement d'une ou plusieurs parties de ces éléments.

L'extrait peut-être brut ou être issu d'un processus d'extraction utilisé de manière habituelle dans le domaine des compléments alimentaires.

De tels processus d'extraction peuvent notamment comprendre une déshydratation ou un séchage à froid de sorte que la partie comestible soit transformée en format adapté pour la consommation par un humain. Les processus peuvent en outre comprendre le broyage ou le découpage de certaines parties des plantes. Des extraits de plantes peuvent ainsi se retrouver sous forme de poudre ou de granulés notamment. Les processus peuvent en outre comprendre le lavage, la désinfection, la décoloration, le séchage et/ou la cuisson de parties de plantes. Des extraits de plantes peuvent donc également se retrouver par exemple sous forme de suspension, de solution aqueuse ou de dispersion. Des extraits peuvent en outre se présenter sous forme de "totum de plante". Un totum de plante correspond à une plante entière, ou une partie de plante, déshydratée par séchage et broyée.

On désigne par "extrait comestible", la ou les parties comestibles de la plante, transformées ou non par un processus d'extraction, présentes dans la composition de l'invention.

Dans des modes de réalisation, la composition de l'invention comprend des particules solides d'extraits de plantes. La taille moyenne du diamètre de particule est généralement inférieure à 500 µm, de préférence inférieure à 400 µm, plus préférentiellement inférieur à 300 µm, plus préférentiellement encore inférieur à 150 µm. Dans un mode de réalisation préférentiel la taille moyenne du diamètre de particules est comprise entre environ 180 µm et environ 90 µm. La taille de particules est exprimée en distribution d90, c'est-à-dire que 90% des particules ont un diamètre inférieur à la valeur déterminée par le granulomètre.

Dans des modes de réalisation de l'invention, la composition peut comprendre des fibres alimentaires. Par exemple la composition peut comprendre des polysaccharides tels que des polysaccharides autres que l'amidon. Ces types de polysaccharides ne sont généralement pas dégradés dans le tract gastro-intestinal. Ceci est au moins partiellement dû à l'incapacité du corps humain d'hydrolyser ces polysaccharides. Un avantage d'inclure des fibres alimentaires dans la composition de l'invention réside dans le fait que ces fibres sont généralement capables de se lier à des graisses alimentaires présentes dans le tract gastro-intestinal et de les évacuer du corps humain sous forme de complexe fibres/graisses via les excréments. Ceci peut contrevenir en partie à l'assimilation et l'absorption de graisses alimentaires par le corps.

Dans d'autres modes de réalisation de l'invention, la composition peut comprendre des fibres alimentaires plus ou moins solubles. À titre d'exemple on peut citer le chitosan, de la gomme acacia, de la gomme de guar, la pectine, les flocons d'avoine, du blé, des fibres de soja, des extraits de betterave, la cellulose, ou tout dérivé de ceux-ci.

Dans encore d'autres modes de réalisation de l'invention, la composition de l'invention peut être couplée avec des agents satiétogènes, des produits drainants, des produits inhibiteurs des enzymes de digestion, des produits prométaboliques, des produits probiotiques, ou des produits prébiotiques. La composition de l'invention peut être qualifiée de produit phytoactif. Elle peut en outre être couplée à d'autres produits phytoactifs tels que des polyphénols ou des terpènes. Une fonction principale de l'invention est d'agir en tant que produit de modulation de tissu adipeux.

La composition de l'invention peut se présenter sous différentes formes adaptées à l'ingestion par voie orale. En particulier la composition peut se présenter sous forme de comprimés, de gélules, de capsules, de poudre, de liquide ou encore sous forme de sirop.

Ainsi, la composition de l'invention peut comprendre des substances véhicule ou de support (*carrier* en anglais) pharmaceutiquement acceptable et/ou compatible avec le marché agroalimentaire. La composition peut comprendre en outre des ingrédients de type diluant, adjuvant, excipient, agent de conservation, charge, agent de désintégration, agent mouillant, émulsifiant, agent de suspension, agent d'intensifiant de goût, agent aromatique, agent odorant, agent antibactérien, agents anti levure, agent lubrifiant et agent de dispersion. Des techniques de formulation sont notamment décrits dans l'ouvrage Remington: The Science and Practice of Pharmacy, 19th Edition, ISBN-13: 978-0912734040 ou dans l'ouvrage Conception des compléments alimentaires : Marché, développement, réglementation et efficacité, ISBN-13: 978-2743022211.

Lorsque la composition de l'invention est sous forme solide ou semi-solide (y compris sous forme de gomme), la substance véhicule peut être du dicalcium phosphate. Les diluants et charges peuvent comporter de l'amidon, du lactose, du sucrose, du glucose, du fructose, du mannitol, de la maltodextrine ou de la cellulose microcristalline. Les agents liants peuvent comporter des agents de type hydroxylepropylcellulose, hydroxylepropylméthylcellulose, carboxyméthylcellulose, gélatine, polyvinyle pyrolidone, polyvinyle acétate, sucrose et gomme acacia. Les agents désintégrants peuvent comporter des agents de type amidon, sodium amidon glycoate, carbonate de calcium, acide alginique ou silicates. Les lubrifiants peuvent comporter du talc, du stéarate de calcium, du stéarate de magnésium, de l'acide stéarique, du sodium sulfate, de la silice ou du sodium fumarate. Les solubilisants peuvent comporter du laurylsulfate de sodium.

Lorsque la composition est par exemple sous forme de comprimés, de gélules ou de capsules, un relâchement contrôlé dans le temps du principe actif peut être prévu. Le profil de relâchement contrôlé peut être adapté selon les besoins visés, et ce notamment au moyen de polymères synthétiques et/ou naturelles. Par ailleurs, un relâchement contrôlé peut-être obtenu au moyen de microsphères ou de liposomes.

Lorsque la composition de l'invention est sous forme liquide ou sous forme de sirop, y compris les solutions de type suspension ou émulsion, elle peut comprendre de l'eau, du propylène glycol, ou de l'alcool. Il est possible d'inclure un humectant tel que le glycérol, ou des agents édulcorants tels que du glucose liquide, de l'aspartame ou du stevia.

La composition de l'invention est tout particulièrement destinée au marché des compléments alimentaires. La composition de l'invention est également destinée au marché pharmaceutique. La composition est en outre destinée au marché des formulations alimentaires.

En particulier, l'invention se rapporte à une composition de produit alimentaire *(foodstuff* en anglais), de complément alimentaire *(food supplement* en anglais), de complément diététique *(dietary supplement* en anglais), de substitut de repas *(meal replacement product* en anglais), de boisson *(beverage* en anglais), de supplément de boisson *(beverage supplement* en anglais), et de produit pharmaceutique *(pharmaceutical product* en anglais) sous toute forme usuelle.

La composition de l'invention peut être incluse dans des plats préparés. Dans des modes de réalisation, ces repas préparés comprennent 0,001 % à 50 % en poids de la composition de l'invention. Dans des modes de réalisations particuliers, les plats préparés comprennent 0,001 % à 40 % en poids, 0,001 % à 30 % en poids, 0,001 % à 20 % en poids, 0,001 % à 10 % en poids, 000,1 % à 5 % en poids ou 000,1 % à 2 % en poids de la composition de l'invention. Avantageusement, les plats préparés comprennent au moins 0,1 % à 5 % en poids ou plus de 5 % en poids de la composition de l'invention. À titre d'exemple, on peut citer des plats préparés tels que des plats cuisinés à réchauffer, des barres céréales, des confiseries, des boissons y compris des boissons de type boisson rapide *(shot drink* en anglais), des snacks ou des gâteaux secs.

La composition de l'invention peut être administrée *per os à* tout moment pour développer ses effets. Elle peut aussi être prise au moment des repas quotidiens en vue de contrôler le poids corporel. Dans des modes de réalisation, l'ingestion de la composition est effectuée 15 à 30 minutes avant ou après le repas.

Le dosage de la composition de l'invention dépend sensiblement du sujet qui l'ingère. Ainsi pour des applications thérapeutiques, le dosage de la composition varie en particulier du poids et de l'indice de masse corporelle (BMI) du sujet. La détermination du dosage peut être déterminée par l'homme du métier.

En général le dosage de la composition peut atteindre jusqu'à 10 g par jour. Dans des modes de réalisation, la formulation de la composition de l'invention est adaptée pour permettre un dosage journalier de 100 mg à environ 7,5 g. Dans d'autres modes de réalisation, la formulation est adaptée pour permettre un dosage journalier de 200 mg à 5 g ou de 300 mg à 3 g. Typiquement, un dosage journalier de 100 mg est prévu.

La composition de l'invention comprend un extrait ou une combinaison d'extraits de plantes. Selon un mode de réalisation de l'invention, la composition comprend au moins 1 % en poids de l'extrait ou de la combinaison d'extraits de plantes. Dans un autre mode de réalisation, la composition comporte 1 à 99 % en poids de l'extrait ou de la combinaison d'extraits de plantes. Dans d'autres modes de réalisation, la composition comprend au moins 5 % en poids, 10 % en poids, 20 % en poids, 30 % en poids, 40 % en poids, 50 % en poids, 60 % en poids, 70 % en poids, 80 % en poids ou au moins 90 % en poids de l'extrait ou de la combinaison d'extraits de plantes.

La composition de l'invention peut en outre comprendre différents types de nutriments tels que des vitamines et/ou des minéraux. Les vitamines peuvent notamment être choisies parmi la vitamine A, la vitamine D, la vitamine E, la vitamine K, la thiamine, la riboflavine, la vitamine B12, les caroténoïdes tels que le bêtacarotène est la zéaxanthine, l'acide folique, la vitamine B8, la vitamine C, ou une combinaison de celles-ci. Les minéraux peuvent notamment être choisis parmi du calcium, du magnésium, du fer, du zinc, du manganèse, du cuivre, de l'iode, du potassium, du molybdène, du sodium, du sélénium, du fluor ou du chlore. Selon des modes de réalisation, les nutriments peuvent être présents dans la composition allant jusqu'à une teneur d'environ 90% ou 50 % en poids.

Le conditionnement ou la formulation de la composition de compléments alimentaires selon l'invention peut être réalisé selon les procédés connus dans l'industrie agroalimentaire. Ces procédés peuvent comprendre notamment des étapes de mixage, de cuisson, d'extrusion, de fermentation, de moulage, de pressage, de séchage, et de mise en forme.

Dans des modes de réalisation, un procédé de préparation de la composition comprend les étapes suivantes :
- lavage d'une partie comestible de plante ;
- découpage de la partie comestible ;
- séchage de la partie comestible ;
- broyage de la partie comestible de sorte à obtenir une poudre.

Un procédé de préparation peut également comprendre des opérations de type extraction solide/liquide, purification liquide/liquide, purification par résine d'absorption, purification membranaire, filtration, concentration, pasteurisation, et/ou séchage par atomisation, sous vide ou encore lyophilisation.

La Demanderesse a mis au point une analyse en deux étapes pour évaluer l'effet de produits provenant de plantes (ou dérivés de plantes) sur du tissu adipeux, et en particulier l'effet de ces produits sur la thermogenèse.

La 1^{ère} étape comporte principalement une étude de biodisponibilité de produits, ou leurs combinaisons, sur des cellules dénommées Caco-2. Dans cette étape, on isole notamment la fraction du produit (ou la partie moléculaire) qui est assimilée par l'organisme humain après une administration *per os.* Par exemple, lorsqu'un produit comporte un principe actif il peut s'agir de la fraction intacte de ce principe actif qui atteint la circulation sanguine générale. Dans d'autres exemples, cette fraction peut consister en l'association de molécules issues du produit, ou peut être constituée de molécules ayant subi une transformation biochimique ou métabolisation cellulaire.

La 2^{ème} étape comporte en particulier une étude de l'effet de la fraction biodisponible sur des préadipocytes humains ou adipocytes humains.

L'utilisation de la fraction biodisponible sur les préadipocytes et adipocytes rend l'évaluation significative sur le plan biologique. En particulier, elle augmente drastiquement la représentativité *in vivo.*

Pour réaliser la 1^{ère} étape la Demanderesse s'est mise à profit un test basé sur l'utilisation des cellules Caco-2.

Les cellules Caco-2 constituent une lignée cellulaire tumorale (cellules immortalisées) humaine d'origine intestinale isolée d'un adénocarcinome colique.

Dans certaines conditions de culture, ces cellules ont la capacité de se différencier spontanément en cellules intestinales organisées en monocouche contigu et polarisées pour former un épithélium mimant une barrière intestinale fonctionnelle. L'épithélium peut notamment présenter des microvillosités, des jonctions serrées, des transporteurs spécifiques au système intestinal, et des enzymes du processus de métabolisation, cf. Pinto M. et al., Enterocyte-like differentiation and polarization of the human colon carcinoma cell line CACO-2 in culture, Biol Cell, 1983, 47:323-30, et Hidalgo IJ. et al., Characterization of the human colon carcinoma cell line (Caco-2) as a model system for intestinal epithelial permeability, Gastroenterology, 1989, 96(3):736-49.

Des essais sur cellules Caco-2 permettent ainsi d'évaluer la perméabilité intestinale d'un produit. Autrement dit, des essais sur Caco-2 renseignent sur la sa capacité d'un composé à traverser la paroi intestinale et, le cas échéant sur sa transformation biochimique, avant de rejoindre la circulation sanguine et lymphatique et se distribuer dans l'organisme.

Ici un test Caco-2, dit essai de perméabilité, est utilisé pour obtenir les fractions biodisponibles des extraits de plantes de l'invention. Il s'agit d'une mise œuvre sur insert de monocouche à compartiment isolé.

Pour cela, des cellules Caco-2 sont cultivées sur une membrane microporeuse placée dans des chambres de culture individuelles.

Après une période de culture d'environ 20 à 25 jours, les cellules forment une monocouche différenciée isolant les compartiments supérieurs et inférieurs de la chambre de culture.

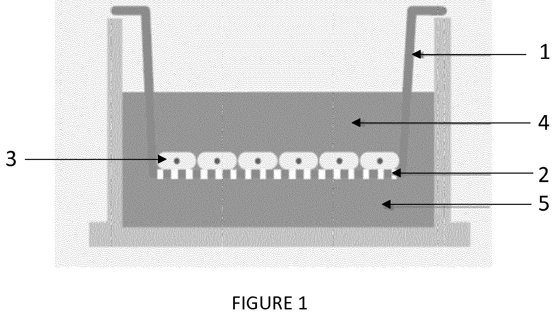

La figure 1 montre un schéma d'un système de test de perméabilité intestinale comportant une culture cellulaire Caco-2. Il s'agit d'un test permettant d'étudier biodisponibilité au travers d'épithélium intestinal.

Le système comporte un insert 1 de Transwell^{®} disponible auprès de la société Merck. L'insert comprend une membrane microporeuse 2 sur laquelle des cellules Caco-2 3 sont cultivées en monocouche. L'ensemble membrane microporeuse / cellules Caco-2 sépare un compartiment apical 4 et un compartiment basolatéral 5.

Ici, la membrane microporeuse est agencée dans six puits. Chaque puit présente un diamètre de 24 mm et un fond à membrane microporeuse présentant des pores de 0,4 µm. Chaque puit est inoculé avec une densité cellulaire de 25x10⁴ cellules/puit. Le milieu de culture est changé chaque jour pendant 21 jours pour obtenir la monocouche cellulaire, cf. Reboul et al., Lutein transport by Caco-2 TC-7 cells occurs partly by a facilitated process involving the scavenger receptor class B type I (SR-BI), Biochem J, 2005, 387(Pt 2):455-61.

Les compartiments apical et basolatéral sont emplis respectivement de 1,5 ml et 2,5 ml de milieu complet. Une exposition des cellules aux extraits de plantes est réalisée pendant une durée d'environ 24 heures. Les cellules et les milieux de culture respectifs des compartiments apical et basolatéral sont récoltés et analysés. Optionnellement, les cellules et les milieux de culture respectifs des compartiments apical (milieu apical) et basolatéral (milieu basolatéral) sont congelés à -80°C pour des expériences ultérieures.

L'utilisation d'un tel système permet d'avoir un accès au côté apical (côté supérieur aux cellules, mimant la lumière intestinale) et au côté basal (côté inférieur aux cellules, mimant le milieu intérieur) de l'épithélium intestinal. Cela permet l'évaluation des échanges de produits entre les deux côtés (apical/basal). Ainsi de manière générale, l'essai de perméabilité sur cellules Caco-2 permet d'investiguer notamment :
- la perméabilité de l'épithélium intestinal vis-à-vis d'un produit d'intérêt ;
- la biodisponibilité attendue chez l'humain ;
- le criblage *(screening* en anglais) et la sélection de produits d'intérêts ;
- la cinétique d'absorption du système intestinal ; et
- l'étude biologique des mécanismes d'absorption.

Après exposition des cellules Caco-2 à des extraits ou des combinaisons d'extraits de plantes introduits dans le milieu apical, il est alors possible de récolter le milieu basolatéral (BM, *basolateral media* en anglais) comprenant la fraction biodisponible, des extraits ou de leurs combinaisons. On notera que le BM peut en outre contenir des métabolites résultants de l'exposition spécifique des cellules Caco-2 aux extraits de plantes. Le BM est généralement récolté et stabilisé par congélation -80°C.

La Demanderesse a ainsi évalué la biodisponibilité d'une multitude d'extraits de plantes afin d'évaluer ensuite l'effet des fractions biodisponibles sur des préadipocytes ou des adipocytes matures.

L'évaluation de l'effet de chaque fraction biodisponible comporte notamment :
**(I)**une étude de cytotoxicité ;
**(II)**une étude de différenciation de préadipocytes et d'accumulation lipidique (adipogenèse) ;
**(III)**une étude d'orientation phénotypique du tissu adipeux (thermogenèse) ;
**(VI)**une étude de métabolisme énergétique du tissu adipeux ; et
**(V)**une étude de lipolyse.

Chaque évaluation est accompagnée d'un ou plusieurs échantillons témoins pour analyser les effets.

### (I) ÉTUDE DE CYTOTOXICITÉ

On parle de cytotoxicité (ou d'un effet cytotoxique) lorsqu'une substance a un effet toxique sur des cellules. Plus particulièrement, la cytotoxicité est la propriété d'un agent chimique ou biologique à être toxique pour les cellules, éventuellement jusqu'à les détruire.

Dans un premier temps (I.1), la Demanderesse a réalisé une pré-étude sur la cytotoxicité d'extraits de plantes sur des cellules Caco-2. Dans un deuxième temps (I.2), la Demanderesse a évalué la cytotoxicité d'extraits biodisponibles de plantes et leurs combinaisons sur des préadipocytes.

### (I.1) ÉTUDE DE CYTOTOXICITÉ D'EXTRAITS DE PLANTES SUR DES CELLULES CACO-2

La Demanderesse a évalué les effets cytotoxiques d'une multitude d'extraits de plantes sur des cellules Caco-2.

L'évaluation comporte une analyse de dommages membranaires, une analyse morphologique de cellules et une estimation de mortalité par coloration au bleu de trypan de cellules mortes.

L'analyse du dommage des membranes cellulaires est réalisée avec le kit de test *CytoTox 96*^{®} *Non-Radioactive Cytotoxicity Assay* disponible auprès de la société *Promega* sous la référence G1780.

Ce test est basé sur le principe que des cellules ayant une membrane cellulaire endommagée libèrent du lactate déshydrogénase (LDH) dans le milieu de culture. Le LDH est mesuré en fournissant dans le milieu du lactate, du nicotinamide adénine dinucléotide oxydé (NAD+) et de l'iodonitrotetrazolium violet en tant que substrats en présence de diaphorase. Il se crée alors du formazan rouge. Le formazan rouge est proportionnel au LDH libéré par les cellules.

Ici, des cellules Caco-2 sont inoculées à une densité de 25x10³ cellules/puits dans des plaques microtitre à 96 puits. Le milieu est changé chaque jour pendant 21 jours pour obtenir une monocouche de cellules, cf. Reboul et al., Lutein transport by Caco-2 TC-7 cells occurs partly by a facilitated process involving the scavenger receptor class B type I (SR-BI), Biochem J, 2005, 387(Pt 2):455-61. Les cellules sont ensuite incubées avec des concentrations d'extraits (variables selon les extraits choisis) dans 2% de milieu à sérum de veau fœtal pendant une durée de 48 heures. Le milieu est changé après 24 heures. Après incubation, le milieu de culture cellulaire est récolté et le taux de LDH est quantifié.

### (I.2) ÉTUDE DE CYTOTOXICITÉ D'EXTRAITS BIODISPONIBLES DE PLANTES SUR DES PRÉADIPOCYTES HUMAINS

La Demanderesse a évalué les effets cytotoxiques d'une multitude d'extraits biodisponibles de plantes, ainsi que les effets cytotoxiques de la combinaison de ces extraits, sur des préadipocytes provenant de différents donneurs.

Les extraits sont récoltés ou dérivés des parties comestibles des plantes respectives.

L'effet cytotoxique des extraits de plantes biodisponibles et leur combinaisons a été évalué sur des préadipocytes. Deux approches techniques ont été mises en œuvre :
- l'évaluation de la viabilité cellulaire par la mesure de la production de formazane par des cellules actives (donc vivantes) ; et
- l'analyse du dommage des membranes cellulaires par la mesure de lactate déshydrogénase (LDH) dans du milieu de culture cellulaire.

L'évaluation de la viabilité cellulaire est réalisée avec le kit de test *CellTiter 96*^{®} *AQueous One Solution Cell Proliferation Assay* disponible auprès de la société *Promega* sous la référence G3580.

Dans ce test, le composé MTS-tetrazolium (3-(4,5-dimethylthiazol-2-yl)-5-(3-carboxymethoxyphenyl)-2-(4-sulfophenyl)-2H-tetrazolium) est transformé par des cellules actives (vivantes) dans un milieu de culture en du formazan coloré. La transformation du MTS-tetrazolium est réalisée par les mitochondries dans les cellules. Le formazan est soluble dans le milieu de culture. La quantité du formazan est directement proportionnelle au nombre de cellules vivantes et peut être mesurée par spectrophotométrie (absorbance).

En pratique, le test de viabilité cellulaire se base donc sur l'activité mitochondriale des cellules.

Il s'ensuit que l'étude de viabilité peut fournir des informations précieuses pour l'étude de métabolisme énergétique (IV). En effet, lorsque les cellules sont viables, ce test renseigne sur l'activité mitochondriale des cellules.

L'analyse du dommage des membranes cellulaires est réalisée avec le kit de test *CytoTox 96*^{®} *Non-Radioactive Cytotoxicity Assay* disponible auprès de la société *Promega* sous la référence G1780 (voir ci-dessus I.1).

Pour évaluer la cytotoxicité d'un extrait de plante ou d'une combinaison d'extraits de plantes sur les cellules, l'extrait ou la combinaison d'extraits est comparé(e) à un témoin.

Le témoin peut être constitué des cellules qui n'ont pas été exposées à un extrait de plante. Le témoin peut également être constitué des cellules qui ont été exposées à un extrait de plante connu non-cytotoxique.

Un extrait est considéré comme cytotoxique si le taux de LDH mesuré est supérieur ou égal à 20% par rapport au taux de LDH mesuré avec l'extrait témoin.

Une analyse statistique est réalisée. Les résultats sont exprimés en pourcentages par rapport à l'extrait témoin ; à l'exception pour le test LDH, pour lequel les résultats sont exprimés comme pourcentages du témoin de lyse cellulaire (moyenne +/- SEM), qui représente le contrôle positif pour la cytotoxicité (le contrôle est fixé à 100%). Des analyses statistiques sont effectuées en utilisant le logiciel *Prism* disponible auprès de la société *GraphPad Software.*

La Demanderesse a obtenu des dons de tissus adipeux, à chaque fois, de trois donneurs différents. Pour une analyse statistique suffisamment représentative, une utilisation de valeurs triples a été mise en œuvre par expérience (pour une expression en erreur type ou SEM - *Standard Error of Mean* en anglais). Ceci résulte en un total de neuf valeurs pour chaque expérience. Des comparaisons entre le traitement avec extrait(s) de plante(s) et les témoins (sans traitement avec extrait) ont été analysées avec le test de Friedman. Ceci permet de comparer des données répétées pour des échantillons dépendants. Le test de Friedman est un test non-paramétrique qui peut être utilisé lorsque l'on est en présence de n échantillons appariés correspondant à n traitements portant sur le même type de cellules, afin de mettre en évidence une différence entre les traitements.

Le test de Friedman est suivi d'un test de Wilcoxon *(Wilcoxon-Mann-Whitney)* pour comparer des échantillons appariés, deux par deux. Des différences ont été considérées comme statistiquement significatives lorsque p ≤ 0.05 (*p ≤ 0.05, **p ≤ 0.01, ***p ≤ 0.001, ****p ≤ 0.0001), p étant la valeur-p *(p-value* ou *probability value* en anglais).

Des préadipocytes provenant d'humain sont utilisés pour l'évaluation de la cytotoxicité des extraits de plantes ou de la combinaison de deux ou plus desdits extraits.

Les trois donneurs de tissus adipeux sont des femmes adultes âgées respectivement de 27, 32 et 34 ans subissant une intervention de chirurgie esthétique ou reconstructive. Les donneurs présentaient un indice de masse corporelle (IMC, ou BMI pour *Body Mass Index* en anglais) respectif de 23,3, 26,7 et 29,3.

Les préadipocytes sont préparés à partir d'une digestion enzymatique d'échantillons du tissu adipeux sous-cutané des donneurs, suivi d'une centrifugation et le prélèvement de la pastille de centrifugation *(cellular pellet* en anglais)
Les préadipocytes sont ensuite étalés à basse densité sur plaque microtitre 96 et incubés pendant 5 à 6 jours en combinaison avec un milieu basolatéral (BM, *basolateral media* en anglais) de cellules Caco-2 exposées à des extraits ou des combinaisons d'extraits, à une dilution 1:8 chacun dans un milieu comprenant 1% de sérum de veau fœtal (FBS). Il s'ensuit une dilution 1:4 du milieu basolatéral total dans le milieu de culture.

La dilution de 1:8 est réalisée pour palier une activité biochimique inhérente au milieu basolatéral vierge, c'est-à-dire un milieu basolatéral dont les cellules Caco-2 n'ont pas été exposées à un extrait de plante. Une dilution de 1:8 permet en effet de diffuser quasi totalement l'activité propre au milieu basolatéral vierge. Les mesures sont ainsi plus sensibles et représentatives.

Le milieu de culture est changé toutes les 48 heures. Chaque ancien milieu de culture est prélevé et récolté pour l'analyse de cytotoxicité.

La viabilité cellulaire est mesurée directement dans les puits de la plaque microtitre.

Ici, les différents milieux basolatéraux de la pré-étude (I.1) sont utilisés. Ces milieux comportent à chaque fois la fraction biodisponible des extraits respectifs. Le mélange de différents milieux basolatéraux permet de tester la combinaison de fractions biodisponibles de différents extraits.

Les concentrations retenues n'ont aucune activité cytotoxique envers les cellules.

### (II) ÉTUDE DE DIFFÉRENTIATION DE PRÉADIPOCYTES ET D'ACCUMULATION LIPIDIQUE (ADIPOGENÈSE)

La Demanderesse a évalué les effets d'une multitude d'extraits biodisponibles de plantes, ainsi que les effets de la combinaison de ces extraits, sur la différenciation de préadipocytes et sur l'accumulation lipidique. Les préadipocytes proviennent de différents donneurs.

Les extraits sont récoltés ou dérivés des parties comestibles des plantes respectives.

Dans des patients atteints de surpoids ou d'obésité on constate une augmentation de la masse du tissu adipeux. Ceci est notamment dû à l'augmentation en taille des cellules formant ce tissu. Plus précisément, ce sont les préadipocytes présents dans le tissu adipeux qui sont responsables par le biais de l'adipogenèse de l'augmentation du nombre d'adipocytes. L'adipogenèse est le processus de différenciation cellulaire dans lequel des préadipocytes deviennent des adipocytes. Les préadipocytes sont des cellules de type fibroblaste *(fibroblast-like cells* en anglais) tandis que les adipocytes sont des cellules volumineuses de forme généralement ronde comportant une goutte de lipide relativement volumineuse, cf. Lowe C.E. et al., "Adipogenesis at a glance", J Cell Sci, 2011, 124:2681-2686.

En pratique, les effets des extraits biodisponibles sur l'accumulation lipidique sont étudiés en mesurant l'accumulation des gouttes de lipides dans les préadipocytes différenciés, c'est-à-dire dans les adipocytes. La mesure est optique et est réalisée par fluorescence.

L'analyse statistique est réalisée dans les mêmes conditions que pour l'étude de cytotoxicité (I).

Des préadipocytes provenant d'humain sont utilisés pour l'étude de l'accumulation lipidique.

Les trois donneurs de tissus adipeux sont des femmes adultes âgées respectivement de 26, 41 et 45 ans subissant une intervention de chirurgie esthétique ou reconstructive. Les donneurs présentaient un indice de masse corporelle (IMC, ou BMI pour *Body Mass Index* en anglais) respectif de 22,2, 22,9 et 26,8.

Les préadipocytes sont isolés par digestion et centrifugation conformément au protocole décrit pour l'étude de cytotoxicité (I) ci-dessus.

Les préadipocytes sont étalés à haute densité sur plaque microtitre 96 et incubés avec un milieu basolatéral de cellules Caco-2 exposées à des extraits ou des combinaisons d'extraits pendant une durée d'environ 10 à 12 jours, cf. Hauner H., Skurk T. and Wabitsch M., "Cultures of Human Adipose Precursor Cells", Method in Molecular Biology, 2001, 155:239-247. Chaque lot de milieu basolatéral exposé à un ou plusieurs extraits est dilué à raison de 1:8 dans du milieu de différenciation avant la mise en contact avec les préadipocytes. Cette dilution est effectué pour palier l'activité du milieu basolatéral vierge (voir ci-dessus). Le milieu de culture est changé toutes les 48 heures (remise en contact avec le BM). Après la culture, les cellules différenciées sont fixées avec une solution à 4% de paraformaldéhyde puis colorées avec du bore-dipyrométhene (BODIPY^{®} disponible auprès de la société *ThermoFisher Scientific* sous la référence D3922) et du 4',6-diamidino-2-phénylindole (DAPI Invitrogen Molecular Probes^{®} disponible auprès de la société *ThermoFisher Scientific* sous la référence D1306). La coloration permet en particulier l'observation des gouttes de lipide et du noyau cellulaire.

La quantification est basée sur des mesures d'intensité de fluorescence sur des images. L'acquisition et le traitement des images comporte notamment :
- l'acquisition de microphotographies avec un vidéo-microscope utilisant la technologie dite Apotome (7 micrographes par puits, 3 puits par condition de traitement) ;
- la quantification de l'accumulation lipidique par le traitement et l'analyse d'image du logiciel ImageJ développé par *National Institutes of Health* (NIH) ;
- la détermination du nombre de noyaux au moyen d'un algorithme de détection par formes et de tri par formes basés sur la taille de noyau et la circularité du noyau ;
- la mise en relation entre la fluorescence détectée et le nombre de noyaux.

### (III) ÉTUDE DE L'ORIENTATION PHÉNOTYPIQUE DU TISSU ADIPEUX (THERMOGENÈSE)

La Demanderesse a évalué les effets d'une multitude d'extraits biodisponibles de plantes, ainsi que les effets de la combinaison de ces extraits, sur la différenciation de préadipocytes ou adipocytes en adipocytes beiges. Plus généralement la Demanderesse a évalué l'orientation d'un phénotype blanc vers un phénotype beige.

Le tissu adipeux comporte deux types différents : le tissu adipeux blanc et le tissu adipeux brun.

Le tissu adipeux blanc comprend majoritairement des adipocytes blancs et est impliqué dans le stockage et la mobilisation d'énergie. Le tissu adipeux brun comporte majoritairement des adipocytes bruns et est impliqué dans la thermogenèse.

Le tissu adipeux brun n'est généralement pas prédominant chez les humains adultes. Toutefois, le tissu adipeux blanc peut comporter des adipocytes bruns après une stimulation de thermogenèse de ce tissu, par exemple après une exposition au froid. Ces adipocytes bruns inclus dans le tissu adipeux blanc sont qualifiés d'adipocytes beiges (ou encore d'adipocytes brite venant de l'anglais *brown in white).*

Les adipocytes bruns et beiges présentent des caractéristiques communes. En particuliers, ces adipocytes comportent un grand nombre de mitochondries. Les mitochondries jouent un rôle clé dans le métabolisme énergétique du tissu adipeux. En outre, adipocytes bruns et beiges expriment la protéine découplante appelée thermogénine (UCP1, *uncoupling protein* 1 en anglais). La thermogénine est présente dans les mitochondries et joue un rôle essentiel dans la thermogenèse, cf. Estève D., Boulet N. et al. "Human white and brite adipogenesis is supported by MSCA1 and is impaired by immune cells", Stem Cell, 2015, 33(4):1277-91.

L'analyse statistique est réalisée dans les mêmes conditions que pour l'étude de cytotoxicité (I).

Des préadipocyctes provenant d'humain sont utilisés pour l'étude de différenciation cellulaire. Les trois donneurs sont ceux de l'étude de cytotoxicité (I) ci-dessus.

Les préadipocytes sont isolés par digestion et centrifugation conformément au protocole décrit pour l'étude de cytotoxicité (I) ci-dessus. Les préadipocytes sont étalés à haute densité sur plaque microtitre 96 et cultivés selon le protocole décrit pour l'étude d'accumulation lipidique (II). La mise en contact avec le milieu basolatéral est réalisée selon le protocole décrit pour l'étude d'accumulation lipidique (II).

Après la culture, les cellules différenciées sont fixées avec une solution à 4% de paraformaldéhyde puis colorées avec un anticorps fluorescent anti-UCP1 disponible auprès de la société *Sigma-Aldrich* sous la référence U6382. En outre, les cellules sont colorées par du bore-dipyrométhene (BODIPY^{®} disponible auprès de la société *ThermoFisher Scientific* sous la référence D3922) et du 4',6-diamidino-2-phénylindole (DAPI Invitrogen Molecular Probes^{®} disponible auprès de la société *ThermoFisher Scientific* sous la référence D1306). La coloration permet en particulier l'observation de UCP1, des gouttes de lipide et du noyau cellulaire.

La quantification est basée sur des mesures d'intensité de fluorescence sur des images. L'acquisition et le traitement des images comporte notamment :
- l'acquisition de microphotographies avec un vidéo-microscope utilisant la technologie dite Apotome (7 micrographes par puits, 3 puits par condition de traitement) ;
- la quantification de UCP1 et d'accumulation lipidique par le traitement et l'analyse d'image du logiciel ImageJ ;
- la détermination du nombre de noyaux au moyen d'un algorithme de détection par formes et de tri par formes basés sur la taille de noyau et la circularité du noyau ;
- l'acquisition de données sur l'expression de UCP1 dans les zones fluorescentes par rapport au nombre total de noyaux et au nombre de noyaux des cellules différenciées (UCP1 ne pouvant s'exprimer majoritairement que dans ces dernières).

### (IV) ÉTUDE DE MÉTABOLISME ÉNERGÉTIQUE DU TISSU ADIPEUX

La Demanderesse a évalué les effets d'une multitude d'extraits biodisponibles de plantes, ainsi que les effets de la combinaison de ces extraits, sur le métabolisme énergétique de préadipocytes provenant de différents donneurs.

Chez les humains, la phosphorylation oxydative se déroule au niveau de la membrane des mitochondries. En résumé, il s'agit la phosphorylation de l'ADP en ATP grâce à l'énergie libérée par l'oxydation de donneurs d'électrons par la chaîne respiratoire.

Pour étudier le métabolisme énergétique, la production d'ATP est mesurée dans les préadipocytes différenciés. Ceci renseigne sur la présence de mitochondries et leur activité. Ici, le test Mitochondrial ToxGlo^{®} disponible auprès de la société *Promega* sous la référence G8000 est utilisé.

Le test comporte une mise en contact des préadipocytes avec un réactif de détection d'ATP. Il s'ensuit la lyse cellulaire des préadipocytes et la génération d'un signal luminescent proportionnel à la quantité d'ATP. Le réactif de détection d'ATP comprend de la luciférine, des inhibiteurs d'ATPase et de la luciférase thermostable Ultra-Glo^{®}. Les valeurs sont normalisées avec une quantification DAPI. La Normalisation est faite par rapport à du milieu basolatéral seul.

L'analyse statistique est réalisée dans les mêmes conditions que pour l'étude de cytotoxicité (I).

Des préadipocyctes provenant d'humain sont utilisés pour l'étude de métabolisme énergétique. Les trois donneurs sont ceux de l'étude de cytotoxicité (I) ci-dessus.

Les préadipocytes sont isolés par digestion et centrifugation conformément au protocole décrit pour l'étude de cytotoxicité (I) ci-dessus. Les préadipocytes sont étalés à haute densité sur plaque microtitre 96 et cultivés selon le protocole décrit pour l'étude d'accumulation lipidique (II). La mise en contact avec le milieu basolatéral est réalisée selon le protocole décrit pour l'étude d'accumulation lipidique (II).

La quantification de la synthèse d'ATP est réalisée sur des cellules différenciées.

### (V) ÉTUDE DE LIPOLYSE D'ADIPOCYTES MATURES EXPOSÉS À DES EXTRAITS BIODISPONIBLES DE PLANTES

Le tissu adipeux blanc est une source majeure d'énergie du corps humain. Les adipocytes stockent des triglycérides (également appelés triacylglycérols, triacylglycérides ou TAG). Sous effort corporel, les triglycérides sont transformés en acides gras non estérifiés (NEFA, *non-esterified fatty acids* en anglais) et en glycérol. Ceci permet de relibérer les triglycérides transformés dans les cellules et dans la circulation sanguine.

Ce processus de transformation est appelé lipolyse. La lipolyse est notamment déclenchée par des catécholamines (en particulier par l'adrénaline et la noradrénaline). La lipolyse est en outre régulée par la lipase hormonosensible (HSL, *hormone-sensitive lipase* en anglais).

La quantification de la production de glycérol est réalisée au moyen du kit d'essai *Glycerol Assay Kit* disponible auprès de la société *Randox* sous la référence GY105.

La quantification de la production d'acides gras non estérifiés est réalisée par les kits d'essai *NEFA HR2 - R1 Set, NEFA HR2 - R2 Set* et *NEFA Standard* disponibles auprès de la société *Fujifilm Wako Chemicals Europe* sous les références respectives 434-91795, 436-91995 et 270-77000.

Chacun de ces kits est basé sur la production d'un composé coloré. Ainsi, les concentrations respectives de glycérol et de d'acides gras non estérifiés sont déterminées au moyen de mesures optiques d'absorbance, et la comparaison de ces mesures à des plages d'absorbance pour de concentration de glycérol et de d'acides gras non estérifiés connues. Les valeurs sont normalisées par quantification d'ADN au moyen du kit Quant-iT^{®} PicoGreen^{®} (Invitrogen^{®}) disponible auprès de la société *ThermoFisher Scientific* sous la référence P7589.

L'analyse statistique est réalisée dans les mêmes conditions que pour l'étude de cytotoxicité (I).

Des adipocytes provenant d'humain sont utilisés pour l'étude de lipolyse.

Les trois donneurs de tissus adipeux sont des femmes adultes âgées respectivement de 34, 40 et 41 ans subissant une intervention de chirurgie esthétique ou reconstructive. Les donneurs présentaient un indice de masse corporelle (IMC, ou BMI pour *Body Mass Index* en anglais) respectif de 31,1, 23,3 et 22,9.

Les adipocytes sont préparés à partir de tissu adipeux sous-cutané digéré par collagénase suivi d'une faible centrifugation.

Les adipocytes sont incubés dans une solution tampon de Krebs-Ringer bicarbonate (KRB), parfois contenant de milieu basolatéral de cellules caco-2 exposées avec, soit des concentrations prédéterminées d'extrait(s) (à raison d'une dilution 1:8 chaque), soit avec des produits de référence. Le temps d'incubation est de 2h à une température de 37°C. Le milieu de culture cellulaire est ensuite récolté et congelé à une température de -20°C pour l'utilisation dans les tests de glycérol et NEFA. Les adipocytes sont également récoltés pour la quantification d'ADN.

### APPLICATION DES ÉTUDES I à V SUR DES EXTRAITS ET COMBINAISONS D'EXTRAITS DE PLANTES

La Demanderesse a mené des investigations sur plus de 100 espèces de plantes différentes. Les investigations ont révélé un grand nombre d'informations parmi lesquelles on citera :
- des informations sur la disponibilité industrielle des plantes ;
- des informations sur l'accessibilité de matière première des plantes ;
- des informations sur statut et réglementation en vigueur pour les plantes ; et
- des informations sur le marketing des plantes.

La Demanderesse a retenu 25 espèces de plantes parmi lesquelles on retrouve *Vigna angularis* communément appelée haricot azuki ou haricot rouge du Japon (graine), *Jasminum officinalis* communément appelée jasmin blanc (fleur), ou encore *Aframomum melegueta* communément appelée maniguette (graine broyée).

Dans une première série de tests la Demanderesse a évalué la cytotoxicité et les effets sur la différenciation adipocytaire et l'accumulation lipidique (adipogenèse), le phenotype beige adipocytaire (thermogenèse), le métabolisme énergétique et la lipolyse en relation avec les extraits biodisponibles provenant de ces 25 espèces de plantes.

La Demanderesse a retenu 8 espèces de plantes. Il s'agit des espèces *Nelumbo nucifera* communément appelée lotus, *Mangifera indica* communément appelé Mangue, *Acacia catechu* communément appelée cachoutier, *Commiphora mukul* communément appelée guggul, *Daucus carota* communément appelée carotte, *Cinnamomum verum* communément appelée cannelle de Ceylan, *Rosa canina* communément appelée l'églantier des chiens ou rosier des haies, et *Helichrysum italicum* communément appelée immortelle ou immortelle d'Italie.

Plus particulièrement, il s'agit d'extraits comestibles de ces espèces. Il s'agit notamment de la fleur broyée de lotus, de la peau broyée de mangue, de l'écorce de cachoutier, de l'exsudat broyé de guggul, de la graine de carotte, de la feuille broyée de cannelle de Ceylan, du fruit (cynorhodon) du rosier des chiens et de la partie aérienne de l'immortelle d'Italie.

Les extraits comestibles de l'invention, ou du moins les composés ou molécules constituants ces extraits, peuvent être désignés de principes actifs. Par extension, lorsque les extraits comestibles subissent une ou plusieurs transformations pour être conditionnés ou formulés pour le marché agroalimentaire, on peut désigner ces extraits transformés de principes actifs.

Le fruit de *Rosa canina* est communément appelé cynorhodon ou cynorrhodon. Ce fruit est désigné généralement en botanique comme "faux-fruit". Il provient de la transformation du conceptacle floral du rosier et de l'églantier. Plus généralement il provient des plantes du genre *Rosa,* de la famille des *Rosacées.*

Selon un mode de réalisation de l'invention, l'extrait comestible de *Rosa canina* provient du fruit ou du "faux-fruit" de cette plante.

Selon un mode de réalisation de la description, l'extrait comestible de *Helichrysum italicum* provient de la partie aérienne de cette plante, c'est-à-dire ici l'ensemble tige(s)/fleur(s)/feuille(s).

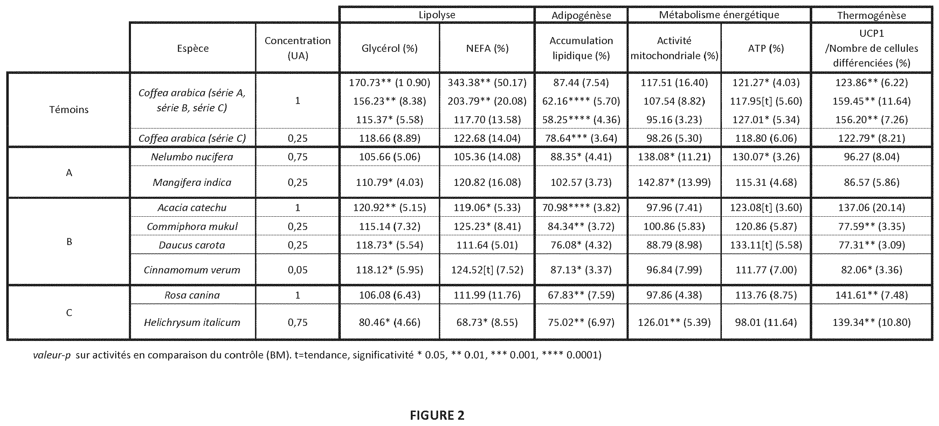

Le tableau de la figure 2 montre les résultats relatifs à la cytotoxicité, la différenciation de préadipocytes et l'accumulation lipidique (adipogenèse), l'orientation phénotypique du tissu adipeux (thermogenèse), le métabolisme énergétique et la lipolyse des extraits biodisponibles de ces huit espèces de plantes.

La première série de tests étaient décomposée en trois sous séries, notées A, B et C dans la figure 2. Chaque série est accompagnée d'extraits témoins provenant de la graine de l'espèce *Coffea arabica* communément appelée café vert, cf. figure 2 respectivement *Coffea arabica* série A, série B et série C.

Plus de précisions relatives aux opérations expérimentales (notamment les techniques chimiques et biochimiques, ou encore l'identité des contrôles positifs et/ou négatifs) pour l'obtention des résultats sont détaillées ci-dessous en rapport avec les combinaisons d'extraits. Les conditions expérimentales sont analogues.

La concentration est indiquée dans la figure 1 en unité arbitraire (UA). Les concentrations respectives des séries A, B et C sont indiquées dans le tableau I ci-dessous.

**Tab.I : Concentrations d'extraits (séries A, B et C).**

| | UA = 0,25 | UA = 0, 5 | UA = 0,75 | UA = 1 |
|---|---|---|---|---|
| Concentration d'extrait série A (g/l) | 0,18 | 0,36 | 0,54 | 0,72 |
| Concentration d'extrait série B (g/l) | 0,18 | 0,36 | 0,54 | 0,72 |
| Concentration d'extrait série C (g/l) | 0,18 | 0,36 | 0,54 | 0,72 |

Les résultats montrent que les extraits sélectionnés ne sont pas cytotoxiques pour les concentrations utilisées dans la première série de tests. Une consommation par des humains ou des animaux est envisageable. L'analyse morphologique de cellules et l'estimation de mortalité par bleu de trypan montrent également des résultats compatibles avec une consommation par des humains ou des animaux.

L'extrait de lotus présente une faible accumulation lipidique et une forte activité sur le métabolisme énergétique.

L'extrait de mangue présente une forte activité lipolytique et une activité sur le métabolisme énergétique (cf. activité mitochondriale).

L'extrait de cachoutier présente une forte activité lipolytique et une faible accumulation lipidique.

L'extrait de guggul présente une forte activité lipolytique et une faible accumulation lipidique.

L'extrait de carotte présente une activité lipolytique et une faible accumulation lipidique.

L'extrait de cannelle de Ceylan présente une forte activité lipolytique et une faible accumulation lipidique.

L'extrait de rose des chiens présente une faible accumulation lipidique et une forte expression d'UCP1 (marqueur de thermogenèse).

L'extrait d'immortelle d'Italie présente une faible accumulation lipidique, une activité sur le métabolisme énergétique (cf. activité mitochondriale), et une forte expression d'UCP1 (marqueur de thermogenèse).

Dans une deuxième série de tests, la Demanderesse a évalué cytotoxicité, l'accumulation lipidique, la différenciation cellulaire, le métabolisme énergétique et la lipolyse des extraits biodisponibles provenant de la combinaison d'une pluralité de ces extraits.

En particulier, la Demanderesse a évalué la cytotoxicité de six combinaisons différentes, chaque combinaison comprenant deux extraits choisis parmi les huit extraits retenus dans la première série de test.

Les combinaisons 1 à 6 sont définies dans le tableau II ci-dessous.

**Tab. II : Combinaison d'extraits de plantes**

| **N° de Combinaison** | **Espèces** |
|---|---|
| 1 | *Acacia catechu* |
| | *Commiphora mukul* |
| 2 | *Acacia catechu* |
| | *Helichrysum italicum* |
| 3 | *Nelumbo nucifera* |
| | *Helichrysum italicum* |
| 4 | *Daucus carota* |
| | *Rosa canina* |
| 5 | *Acacia catechu* |
| | *Rosa canina* |
| 6 | *Daucus carota* |
| | *Cinnamomum verum* |

### RÉSULTATS D'ÉTUDE DE CYTOTOXICITÉ (I) DE COMBINAISONS D'EXTRAITS

Le milieu basolatéral (BM) comprenant les extraits biodisponibles du système à cellules Caco-2 est récolté. Les préadipocytes sont exposés à ce milieu BM pour le test de cytotoxicité. En outre, un contrôle avec des milieux BM respectifs sans extraits est réalisé, ainsi qu'un contrôle de base avec du milieu de base 1% FBS. Un traitement des préadipocytes avec du triton 0,2% est réalisé pour un contrôle de cytotoxicité maximale (100% ; contrôle de lyse). Pour la viabilité, un contrôle positif avec du milieu 10% FBS est en outre réalisé.

Le tableau III montre les résultats de cytotoxicité.

**Tab.III : Cytotoxicité**

| | **Cytotoxicité** |
|---|---|
| | % Contrôle de base (SEM) |
| Contrôle de base | 100.00 (11.76) |
| Contrôle de lyse | 1252.05** (218.83) |
| Contrôle 1-6 | 214.68 (100.70) |
| Contrôle 2-4-5 | 160.19 (75.10) |

| Contrôle 3 | 104.08 (57.49) |
|---|---|
| | **Cytotoxicité** |

| | % Contrôle respectif (SEM) |
|---|---|
| Combinaison 1 | 39.81 (24.64) |
| Combinaison 6 | 40.99 (17.80) |
| Combinaison 2 | 69.59 (22.67) |
| Combinaison 4 | 87.12 (33.18) |
| Combinaison 5 | 77.83 (29.31) |
| Combinaison 3 | 216.99 (81.74) |

Les résultats du tableau III sont exprimés en pourcentage par rapport au contrôle pour la libération de LDH. Les analyses statistiques sont réalisées en mettant en relation les milieux BM comprenant les extraits avec les milieux BM respectifs sans extraits.

Les résultats montrent que les combinaisons ne sont pas cytotoxiques. La combinaison n° 3 montre un pourcentage élevé par rapport aux autres combinaisons. Toutefois, les cellules exposées à cette combinaison n° 3 ne montrent pas de changements significatifs dans leur morphologie. Cette combinaison n'est pas cytotoxique.

Le tableau IV montre les résultats de viabilité.

**Tab.IV : Viabilité**

| | **Activité Mitochondriale** |
|---|---|
| | % Contrôle de base (SEM) |
| Contrôle de base | 100.00 (2.88) |
| Contrôle positif | 129.60** (2.76) |
| Contrôle de lyse | 23.78** (1.14) |
| Contrôle 1-6 | 76.47** (3.29) |
| Contrôle 2-4-5 | 77.54** (2.32) |
| Contrôle 3 | 81.64** (2.23) |

| | **Activité Mitochondriale** |
|---|---|
| | % Contrôle respectif (SEM) |
| Combinaison 1 | 133.88** (2.19) |
| Combinaison 6 | 104.13 (1.50) |
| Combinaison 2 | 129.59** (6.02) |
| Combinaison 4 | 92.28 (2.71) |
| Combinaison 5 | 95.77 (2.79) |
| Combinaison 3 | 105.53 (6.41) |

Les résultats du tableau IV sont exprimés en pourcentage par rapport au contrôle pour l'activité mitochondriale.

En particulier, les combinaisons n° 1 et n° 2 augmentent considérablement l'activité mitochondriale.

Chacune des six combinaisons d'extraits n° 1 à n° 6 montre des résultats compatibles avec une ingestion par l'humain ou l'animal.

### RÉSULTATS D'ÉTUDE DE DIFFÉRENCIATION DE PRÉADIPOCYTES ET D'ACCUMULATION LIPIDIQUES (II) DANS DES CELLULES EXPOSÉES À DES COMBINAISONS D'EXTRAITS BIODISPONIBLES

Le milieu basolatéral (BM) comprenant les extraits biodisponibles du système à cellules Caco-2 est récolté pour le test.

Des préadipocytes non traités par mixture de différenciation (NDIF), ainsi que des préadipocytes traités avec un antagoniste PPARγ (GW9662, disponible auprès de la société Sigma-Aldrich sous la référence M6191 - le GW9662 inhibe la différenciation) à une concentration de 0,1 µM, sont utilisés pour des contrôles négatifs.

Les résultats sont exprimés en pourcentages par rapports aux résultats obtenus pour des milieux BM respectifs sans extraits. Les analyses statistiques sont réalisées en mettant en relation les milieux BM comprenant les extraits avec les milieux BM respectifs sans extraits.

Des microphotographies à fluorescence sont réalisées pour quantifier l'accumulation lipidique (Bodipy^{®}). Des microphotographies à lumière visible sont réalisées pour analyser l'état morphologique des cellules.

Le tableau V montre les résultats relatifs à l'accumulation lipidique.

**Tab. V : Accumulation lipidique**

| | **Accumulation lipidique** |
|---|---|
| | % Différencié (SEM) |
| Non-différencié | 5.45* (2.19) |
| Différencié | 100.00 (18.06) |
| GW9662 | 30.59* (8.25) |
| Contrôle 1-6 | 189.63* (22.78) |
| Contrôle 2-4-5 | 309.96* (13.25) |
| Contrôle 3 | 239.17* (20.24) |

| | **Accumulation lipidique** |
|---|---|
| | % Contrôle respectif (SEM) |
| Combinaison 1 | 73.21t (7.64) |
| Combinaison 6 | 97.90 (7.06) |
| Combinaison 2 | 45.52** (3.82) |
| Combinaison 4 | 57.05** (6.17) |
| Combinaison 5 | 49.57** (6.11) |
| Combinaison 3 | 73.65* (4.57) |

Les combinaisons d'extraits n° 2 à n°5 montrent une tendance remarquable à l'inhibition de l'accumulation lipidique.

### RÉSULTATS D'ÉTUDE D'ORIENTATION PHÉNOTYPIQUE (III) DU TISSUS ADIPEUX (THERMOGENÈSE) DANS DES CELLULES EXPOSÉES À DES COMBINAISONS D'EXTRAITS BIODISPONIBLES.

Le milieu basolatéral (BM) comprenant les extraits biodisponibles, du système à cellules Caco-2 est récolté pour le test.

Les résultats sont exprimés en pourcentages par rapports aux résultats obtenus pour des milieux BM respectifs sans extraits. Les analyses statistiques sont réalisées en mettant en relation les milieux BM comprenant les extraits avec les milieux BM respectifs sans extraits.

Des microphotographies à fluorescence sont réalisées pour quantifier l'expression de UCP1. Des microphotographies à lumière visible sont réalisées pour analyser l'état morphologique des cellules.

Les contrôles négatifs sont réalisés de la même façon que pour l'étude de différenciation de préadipocytes et d'accumulation lipidique (II) ci-dessus (absence NDIF et présence GW9662).

Le tableau VI montre les résultats de l'expression protéique d'UCP1 en normalisés par rapport au nombre total de cellules.

**Tab. VI : Expression UCP1 (normalisation cellules totales)**

| | **UCP1** |
|---|---|
| | % Différencié (SEM) |
| Non-différencié | 77.52 (9.13) |
| Différencié | 100.00 (11.06) |
| GW9662 | 83.38 (11.95) |
| Contrôle 1-6 | 131.29t (10.38) |
| Contrôle 2-4-5 | 153.47t (24.85) |
| Contrôle 3 | 162.70* (8.68) |

| | **UCP1** |
|---|---|
| | % Contrôle respectif (SEM) |
| Combinaison 1 | 100.50 (10.27) |
| Combinaison 6 | 157.76* (19.51) |
| Combinaison 2 | 146.69* (13.67) |
| Combinaison 4 | 168.81* (14.52) |
| Combinaison 5 | 135.54* (12.72) |
| Combinaison 3 | 110.38 (7.76) |

Les combinaisons d' extraits n° 2, n° 4, n° 5 et n° 6 montrent une expression remarquable d'UCP1 dans les préadipocytes.

Le tableau VII montre les résultats de l'expression protéique d'UCP1 normalisés par rapport au nombre total de cellules différenciées.

À titre informatif, ce mode d'expression peut s'avérer judicieux en présence de composés qui peuvent induire une inhibition de la différenciation adipocytaire, car l'UCP1 ne peut s'exprimer majoritairement que dans les adipocytes matures. Ceci permet de différencier l'effet sur l'expression de l'UCP1, de celui sur la différenciation adipocytaire.

**Tab. VII : Expression UCP1 (normalisation cellules différenciées)**

| | **UCP1** |
|---|---|
| | % Différencié (SEM) |
| Différencié | 100.00 (16.19) |
| Contrôle 1-6 | 94.96 (5.90) |
| Contrôle 2-4-5 | 77.46 (10.16) |
| Contrôle 3 | 103.03 (8.24) |

| | **UCP1** |
|---|---|
| | % Contrôle respectif (SEM) |
| Combinaison 1 | 127.22 (18.64) |
| Combinaison 6 | 158.55 (22.67) |
| Combinaison 2 | 253.42** (30.17) |
| Combinaison 4 | 262.13** (33.54) |
| Combinaison 5 | 248.51** (39.69) |
| Combinaison 3 | 127.12 (16.87) |

Les combinaisons d'extraits n° 2, n° 4 et n° 5 montrent une expression remarquable d'UCP1 dans les préadipocytes différenciés.

### RÉSULTATS D'ÉTUDE DE MÉTABOLISME ÉNERGÉTIQUE DU TISSUS ADIPEUX (IV) DANS DES CELLULES EXPOSÉES À DES COMBINAISONS D'EXTRAITS BIODISPONIBLES

Le milieu basolatéral (BM) comprenant les extraits biodisponibles du système à cellules Caco-2 est récolté pour le test.

Un premier contrôle négatif est réalisé de la même façon que pour l'étude de différenciation de préadipocytes et d'accumulation lipidique (II) ci-dessus : absence NDIF. Un deuxième contrôle négatif de production d'ATP est réalisé en présence de la toxine mitochondriale roténone (disponible auprès de la société Sigma-Aldrich sous la référence R8875) à une concentration de 400 nM (pendant une durée de 2 heures sur les cellules différenciées).

Les résultats sont exprimés en pourcentages par rapports aux résultats obtenus pour des milieux BM respectifs sans extraits. Les analyses statistiques sont réalisées en mettant en relation les milieux BM comprenant les extraits avec les milieux BM respectifs sans extraits.

Le tableau VIII montre les résultats de production d'ATP.

**Tab. VIII : Production d'ATP**

| | **Production d'ATP** |
|---|---|
| | % Différencié (SEM) |
| Non-différencié | 85.15 (3.85) |
| Différencié | 100.00 (6.44) |
| Roténone | 27.95** (2.33) |
| Contrôle 1-6 | 181.44** (7.97) |
| Contrôle 2-4-5 | 201.09** (9.90) |
| Contrôle 3 | 203.74** (13.21) |

| | **Production d'ATP** |
|---|---|
| | % Contrôle respectif (SEM) |
| Combinaison 1 | 109.29 (6.40) |
| Combinaison 6 | 96.83 (3.42) |
| Combinaison 2 | 100.20 (3.35) |
| Combinaison 4 | 92.11* (2.60) |
| Combinaison 5 | 102.96 (3.51) |
| Combinaison 3 | 94.20 (3.04) |

Les résultats montrent que la production d'ATP n'est pas significativement altérée pour des cellules exposées aux combinaisons d'extraits n° 1 à n° 6.

### RÉSULTATS D'ÉTUDE DE LIPOLYSE (V) DANS DES CELLULES EXPOSÉES À DES COMBINAISONS D'EXTRAITS BIODISPONIBLES

Le milieu basolatéral (BM) comprenant les extraits biodisponibles du système à cellules Caco-2 est récolté pour le test.

La forskoline (1 µM, disponible auprès de la société Sigma-Aldrich sous la référence F6886) et l'isoprotérénol (0,1 µM, disponible auprès de la société Sigma-Aldrich sous la référence I6504) sont utilisés comme activateurs de lipolyse. La forskoline est un composé provenant de la plante *Coleus forskohlii* provoquant la lipolyse en activant l'adénylate cyclase. L'isoprotérénol est un composé pharmacologique agoniste du récepteur β-adrénergique. L'isoprotérénol provoque la lipolyse en activant les récepteurs β-adrénergiques ce qui mène à l'activation de l'adénylate cyclase. L'adénylate cyclase produit de l'adénosine monophosphate cyclique qui est un messager intracellulaire déclenchant une cascade de réactions biochimique qui mène à l'activation de la lipolyse.

Les résultats sont exprimés en pourcentages par rapports aux résultats obtenus pour des milieux BM respectifs sans extraits. Les analyses statistiques sont réalisées en mettant en relation les milieux BM comprenant les extraits avec les milieux BM respectifs sans extraits.

Le tableau IX montre les résultats relatifs aux mesures de glycérol. Le tableau X montre les résultats relatifs aux mesures de NEFA.

**Tab. IX : Mesures de Glycérol**

| | **Glycérol** |
|---|---|
| | % Différencié (SEM) |
| Contrôle de base | 100.00 (2.02) |
| Forskolin | 151.62** (4.82) |
| Isoprotérénol | 213.97** (17.87) |
| Contrôle 1-6 | 70.81** (4.96) |
| Contrôle 2-4-5 | 77.23** (3.56) |
| Contrôle 3 | 72.27** (6.78) |

| | **Glycérol** |
|---|---|
| | % Contrôle respectif (SEM) |
| Combinaison 1 | 98.26 (4.79) |
| Combinaison 6 | 115.72 (8.29) |
| Combinaison 2 | 74.38** (4.45) |
| Combinaison 4 | 118.24* (7.71) |
| Combinaison 5 | 92.66 (2.75) |
| Combinaison 3 | 86.87 (6.64) |

**Tab. X : Mesures de NEFA**

| | **NEFA** |
|---|---|
| | % Différencié (SEM) |
| Contrôle de base | 100.00 (2.11) |
| Forskolin | 179.70** (12.42) |
| Isoprotérénol | 241.48** (33.73) |
| Contrôle 1-6 | 70.78 (12.44) |
| Contrôle 2-4-5 | 66.87* (8.32) |
| Contrôle 3 | 62.29* (9.34) |

| | **NEFA** |
|---|---|
| | % Contrôle respectif (SEM) |
| Combinaison 1 | 91.20 (8.40) |
| Combinaison 6 | 93.89 (11.07) |
| Combinaison 2 | 35.38** (7.51) |
| Combinaison 4 | 88.38 (7.49) |
| Combinaison 5 | 74.74** (5.56) |
| Combinaison 3 | 51.13* (14.53) |

Les résultats montrent notamment que la combinaison n° 4 augmente la libération de glycérol.

Il s'ensuit de l'ensemble des résultats ci-dessus que les combinaisons n° 1 à n° 6 sont des combinaisons de nature à traiter efficacement des sujets atteints de surpoids ou d'obésité.

Dans une troisième et quatrième série, la Demanderesse s'est intéressée à la combinaison de respectivement trois et quatre extraits biodisponibles choisies parmi des extraits biodisponibles de *Nelumbo nucifera, Mangifera indica, Acacia catechu, Commiphora mukul, Daucus carota, Cinnamomum verum, Rosa canina,* et *Helichrysum italicum.*

Les résultats de la combinaison triple ou quadruple sont prometteurs en ce qui concerne au moins l'un(e) parmi la cytotoxicité, la différenciation de préadipocytes et l'accumulation lipidique (adipogenèse), l'orientation phénotypique du tissu adipeux (thermogenèse), le métabolisme énergétique ou la lipolyse.

Des formulations de la composition comportant trois ou quatre extraits comestibles de plantes choisies parmi *Nelumbo nucifera, Mangifera indica, Acacia catechu, Commiphora mukul, Daucus carota, Cinnamomum verum, Rosa canina,* et *Helichrysum italicum* sont donc prévues.

D'une manière générale, l'une quelconque de la combinaison de 2 des 8 extraits semble présenter un effet synergique si bien que les résultats sont meilleurs pour au moins l'une des évaluations II à V ci-dessus par rapport aux extraits isolés.

En outre, l'une quelconque de la combinaison de 3 ou 4 des 8 extraits semble également présenter un effet synergique si bien que les résultats sont meilleurs pour au moins l'une des évaluations II à V ci-dessus par rapport aux extraits isolés.

Dans un mode particulièrement préféré, la composition comporte l'une quelconque de la combinaison de 3 ou 4 des extraits comestibles de plantes choisies parmi *Acacia catechu, Daucus* carota, *Rosa canina* et *Helichrysum italicum.*

Un avantage particulier de l'invention réside dans le fait que les extraits ou combinaisons d'extraits identifiés par la Demanderesse ont un effet sur la thermogenèse. La composition peut donc être administrée *per os* à tout moment. Une prise rapprochée des repas n'est pas déterminante pour obtenir les effets de la composition de l'invention.

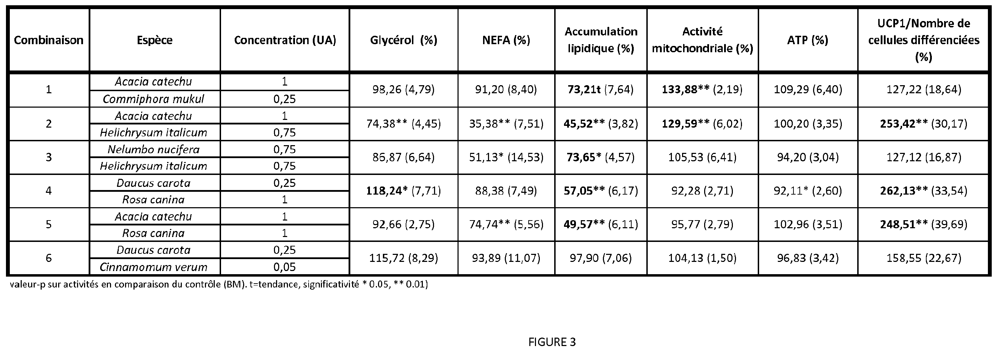

Le tableau de la figure 3 montre une récolte des résultats relatifs à la cytotoxicité, la différenciation de préadipocytes et l'accumulation lipidique (adipogenèse), l'orientation phénotypique du tissu adipeux (thermogenèse), le métabolisme énergétique et la lipolyse des combinaisons d'extraits biodisponibles selon l'invention.

Dans un exemple de réalisation, la composition de complément alimentaire peut se présenter sous forme de comprimé 1 g. Dans cet exemple les teneurs en ingrédients sont les suivantes :
- extrait(s) comestible(s) de plante : 750 à 800 mg ;
- micro cristalline cellulose : 114 à 164 mg ;
- croscarmellose sodium : 40 mg ;
- silicon dioxyde : 10 mg ;
- stéarate de magnésium : 6 mg ;
- enrobage en hydroxypropyl méthylcellulose : 30 mg.

Dans un mode préférentiel, ce comprimé comporte une combinaison de deux extraits comestibles de plante. Dans un mode particulièrement préféré, cette combinaison se compose de 1% à 99%, de préférence 5% à 95%, plus préférentiellement encore 10% à 90% d'un premier extrait et de 1% à 99%, de préférence 5% à 95%, plus préférentiellement encore 10% à 90% d'un deuxième extrait, les premier et deuxième extraits étant choisis parmi *Nelumbo nucifera, Mangifera indica, Acacia catechu, Commiphora mukul, Daucus carota, Cinnamomum verum, Rosa canina,* et *Helichrysum italicum.*

Dans un autre mode de réalisation, le comprimé comporte une combinaison de deux extraits comestibles de plante à raison de 50% chacun.

Dans un mode particulièrement préféré, cette combinaison de deux extraits de plante se compose d'environ 15% à 25% de *Daucus carota* et d'environ 75% à 85% de *Rosa canina.* Plus généralement, la combinaison se compose d'environ 15% à 85% de *Daucus carota* et d'environ 15% à 85% de *Rosa canina.* Par exemple, la combinaison peut se composer d'environ 50% de *Daucus carota* et d'environ 50% de *Rosa canina.*

Dans un autre mode préféré, la combinaison de deux extraits de plante se compose d'environ 15% à 85% de *Acacia catechu* et d'environ 15% à 85% de *Commiphora mukul.* Par exemple, la combinaison peut se composer d'environ 50% de *Acacia catechu* et d'environ 50% de *Commiphora mukul.*

Dans un autre mode particulièrement préféré, la combinaison de deux extraits de plante se compose d'environ 15% à 85% de *Acacia catechu* et d'environ 15% à 85% de *Helichrysum italicum.* Par exemple, la combinaison peut se composer d'environ 50% de *Acacia catechu* et d'environ 50% de *Helichrysum italicum.*

Dans un autre mode particulièrement préféré, la combinaison de deux extraits de plante se compose d'environ 15% à 85% de *Nelumbo nucifera* et d'environ 15% à 85% de *Helichrysum italicum.* Par exemple, la combinaison peut se composer d'environ 50% de *Nelumbo nucifera* et d'environ 50% de *Helichrysum italicum.*

Dans un autre mode particulièrement préféré, la combinaison de deux extraits de plante se compose d'environ 15% à 85% de *Acacia catechu* et d'environ 15% à 85% de *Rosa canina.* Par exemple, la combinaison peut se composer d'environ 50% de *Acacia catechu* et d'environ 50% de *Rosa canina.*

Dans un autre mode particulièrement préféré, la combinaison de deux extraits de plante se compose d'environ 15% à 85% de *Daucus carota* et d'environ 15% à 85% de *Cinnamomum verum.* Par exemple, la combinaison peut se composer d'environ 50% de *Daucus carota* et d'environ 50% de *Cinnamomum verum.*

Les combinaisons d'extraits :
- de *Daucus carota* et de *Rosa canina ;*
- de *Acacia catechu* et de *Helichrysum italicum ;* et
- de *Acacia catechu* et *Rosa canina,*
présentent une forte activité en stimulation de thermogenèse.

Dans un mode particulier, ce comprimé est composé de :
- 250 mg d'extrait comestible de *Daucus carota* (graine) ;
- 550 mg d'extrait comestible de *Rosa canina* (cynorhodon) ;
- micro cristalline cellulose : 114 mg ;
- croscarmellose sodium : 40 mg ;
- silicon dioxyde : 10 mg ;
- stéarate de magnésium : 6 mg ;
- enrobage en hydroxypropyl méthylcellulose : 30 mg

Dans un autre mode de réalisation, ce comprimé est composé de :
- 400 mg d'extrait comestible de *Daucus carota* (graine) ;
- 400 mg d'extrait comestible de *Rosa canina* (cynorhodon) ;
- micro cristalline cellulose : 114 mg ;
- croscarmellose sodium : 40 mg ;
- silicon dioxyde : 10 mg ;
- stéarate de magnésium : 6 mg ;
- enrobage en hydroxypropyl méthylcellulose : 30 mg

Dans un mode de réalisation, la composition de l'invention se présente sous forme de gélule de taille "0". Ce type de gélules a une capacité de charge d'environ 300 à 450 mg. Dans ce mode de réalisation, chaque gélule comprend:
- du stéarate de magnésium lorsqu'il s'agit de poudre/produits secs: 5 à 10 mg ; ou
- de la silice (dioxyde de silicium) lorsqu'il s'agit de poudres grasses : 5 à 10 mg ; et
- environ 300 à 400 mg de principe actif.

Dans un autre mode de réalisation, la composition de l'invention se présente sous forme de comprimé comprenant:
- du stéarate de magnésium lorsqu'il s'agit de poudre/produits secs: 5 à 10 mg ; ou
- de la silice (dioxyde de silicium) lorsqu'il s'agit de poudres grasses : 5 à 10 mg ; et
- du dicalcium phosphate (DCP) : 10 à 80 mg ;
- de la gomme d'acacia ou de la cellulose microcristalline : 200 à 300 mg ; et
- environ 200 à 300 mg de principe actif.

Dans un autre mode de réalisation la composition de l'invention se présente sous forme de gélule (capsule) comprenant :- 300 mg de principe actif ;
- 95 mg d'un mélange d'hydroxyméthylcellulose et chlorophylline ;
- 89,5 mg de farine de grains longs de riz blanc ;
- 4,5 mg de stéarate de magnésium ; et
- 6 mg de concentré de coque de riz.

Plus généralement, des comprimés comprenant la composition de l'invention peuvent comprendre de 1% à 99% de principe actif (extrait(s) de plante(s)) et le reste en excipients et additifs, par exemple de 0 à 10% de stéarate de magnésium et de 0 à 80% de maltodextrine.

Plus généralement, des gélules comprenant la composition de l'invention peuvent comprendre de 1% à 99% de principe actif et le reste en excipients et additifs, par exemple 0 à 10% de silice et de 0 à 80% de maltodextrine.

Plus généralement, des sticks orodispersibles (sachets de poudre non pulvérulente et prêt à être directement ingéré ou préliminairement dilués dans un verre d'eau) ou sticks à gel (sachet à liquide visqueux) comprenant la composition de l'invention peuvent comprendre de 0,1% à 70% de principe actif et le reste en excipients et additifs et éventuellement des agents aromatiques et agents de granulation.

La composition de l'invention peut se présenter sous forme d'oro-films (films fondants sur la langue) ou de poudre à diluer dont la quantité de principe actif peut aller jusqu'à par exemple 100%. La composition de l'invention peut se présenter en outre sous forme de chewing-gum dont la quantité de principe actif peut aller jusqu'à par exemple 50% ou encore sous forme de shot (boisson en petite portion, généralement environ 10 à 25 cl) dont la quantité de principe actif peut aller jusqu'à par exemple 30%.

Plus généralement, la composition peut se présenter sous toute forme usuelle de l'industrie alimentaire ou de l'industrie pharmaceutique.

Dans le cadre d'une exploitation dans le domaine pharmaceutique, la composition peut être définie en particulier comme une composition comprenant un extrait comestible d'au moins une des plantes choisies parmi le groupe constitué de *Nelumbo nucifera, Mangifera indica, Acacia catechu, Commiphora mukul, Daucus carota, Cinnamomum verum, Rosa canina,* et *Helichrysum italicum* pour une utilisation en tant que médicament.

Dans le cadre d'une exploitation dans le domaine médicale, la composition peut être définie en particulier comme une composition comprenant un extrait comestible d'au moins une des plantes choisies parmi le groupe constitué de *Nelumbo nucifera, Mangifera indica, Acacia catechu, Commiphora mukul, Daucus carota, Cinnamomum verum, Rosa canina,* et *Helichrysum italicum* pour une utilisation dans une méthode médicale.

La composition et/ou le produit de l'invention défini dans les revendications permet en particulier d'activer la thermogenèse chez un sujet humain ou animal. Plus généralement, la composition et/ou le produit de l'invention défini dans les revendications peut être qualifié de modulateur de tissu adipeux ou de brûleur de graisse.

En d'autres termes, la composition et les produits qui en découlent sont des modulateurs de tissu adipeux ou brûleurs de graisse, en particulier par l'activation ciblée de la thermogenèse chez un sujet humain ou animal.

## Revendications

1. Composition orale comprenant une combinaison de plantes, ladite combinaison étant une combinaison de la graine de *Daucus carota* et du fruit de *Rosa canina,* ou de leurs extraits obtenus par déshydratation, séchage à froid, extraction solide/liquide, broyage ou découpage, pour l'utilisation dans le traitement et/ou la prévention d'une affection choisie parmi l'obésité et une maladie métabolique chez un humain ou un animal.

2. Composition pour l'utilisation selon la revendication 1, dans laquelle ladite combinaison de plantes est sous forme pulvérulente ou granulaire dont le diamètre granulométrique d90 des particules est inférieur ou égale à 180 µm.

3. Composition pour l'utilisation selon l'une des revendications précédentes, comprenant 0,001% à 99% en poids, de préférence 10% à 80% en poids, de ladite combinaison de plantes.

4. Composition pour l'utilisation selon l'une des revendications précédentes, sous une forme choisie parmi un comprimé, un cachet, une capsule, un granule, une gélule, une dragée, une gomme à mâcher, une pâte, une boisson, un sirop et une poudre.

5. Composition pour l'utilisation selon l'une des revendications précédentes, sous une forme choisie parmi un produit alimentaire, un complément alimentaire, un complément diététique, un substitut de repas, une boisson, un supplément de boisson et un produit pharmaceutique.

6. Composition pour l'utilisation selon l'une des revendications précédentes, sous une forme de produit pharmaceutique, comprenant en outre un ou plusieurs supports et/ou excipients.

7. Procédé non-thérapeutique pour contrôler le poids corporel d'un sujet, ledit procédé comprenant l'administration d'une quantité efficace d'une composition orale comprenant une combinaison de plantes, ladite combinaison étant une combinaison de la graine de *Daucus carota* et du fruit de *Rosa canina,* ou de leurs extraits obtenus par déshydratation, séchage à froid, extraction solide/liquide, broyage ou découpage.

## Patentansprüche

1. Orale Zusammensetzung, die eine Kombination von Pflanzen umfasst, wobei die Kombination eine Kombination aus dem Samen *Daucus carota* und der Frucht von *Rosa canina* oder deren Extrakte ist, die durch Dehydrierung, Kalttrocknung, Fest-/Flüssig-Extraktion, Mahlen oder Schneiden gewonnen werden, zur Verwendung bei der Behandlung und/oder Vorbeugung einer Erkrankung, ausgewählt aus Adipositas und einer Stoffwechselerkrankung, bei Menschen oder Tieren.

2. Zusammensetzung zur Verwendung nach Anspruch 1, wobei die Pflanzenkombination in pulverförmiger oder körniger Form vorliegt, deren Korndurchmesser d90 der Partikel kleiner oder gleich 180 µm ist.

3. Zusammensetzung zur Verwendung nach einem der vorhergehenden Ansprüche, umfassend 0,001 Gew.-% bis 99 Gew.-%, vorzugsweise 10 Gew.-% bis 80 Gew.-%, der Pflanzenkombination.

4. Zusammensetzung zur Verwendung nach einem der vorhergehenden Ansprüche, in einer Form, die aus einer Tablette, einer Pille, einer Kapsel, einem Granulat, einer Hartkapsel, einem Dragee, einem Kaugummi, einer Paste, einem Getränke, einem Sirup und einem Pulver ausgewählt ist.

5. Zusammensetzung zur Verwendung nach einem der vorhergehenden Ansprüche, in einer Form, die aus einem Lebensmittelprodukt, einem Nahrungsergänzungsmittel, einem Diätzusatz, einem Mahlzeitenersatz, einem Getränk, einem Getränkezusatz und einem pharmazeutischen Produkt ausgewählt ist.

6. Zusammensetzung zur Verwendung nach einem der vorhergehenden Ansprüche, in Form eines pharmazeutischen Produkts, das ferner einen oder mehrere Träger und/oder Hilfsstoffe umfasst.

7. Nichttherapeutisches Verfahren zur Kontrolle des Körpergewichts einer Person, wobei das Verfahren die Verabreichung einer wirksamen Menge einer oralen Zusammensetzung umfasst, die eine Kombination von Pflanzen umfasst, wobei die Kombination eine Kombination aus dem Samen *Daucus carota* und der Frucht von *Rosa canina* oder deren Extrakte ist, die durch Dehydrierung, Kalttrocknung, Fest-/Flüssig-Extraktion, Mahlen oder Schneiden gewonnen werden.

## Claims

1. Oral composition comprising a combination of plants, said combination being a combination of *Daucus carota* seed and *Rosa canina* fruit, or extracts thereof obtained by dehydration, cold drying, solid/liquid extraction, grinding or cutting, for use in the treatment and/or prevention of a condition selected from obesity and a metabolic disease in a human or animal.

2. Composition for use according to Claim 1, wherein said combination of plants is in powder or granular form, the particle diameter d90 of the particles being less than or equal to 180 µm.

3. Composition for use according to one of the preceding claims, comprising 0.001% to 99% by weight, preferably 10% to 80% by weight, of said combination of plants.

4. Composition for use according to one of the preceding claims, in a form selected from a tablet, a pill, a capsule, a granule, a softgel, a coated tablet, chewing gum, a paste, a drink, a syrup and a powder.

5. Composition for use according to any one of the preceding claims, in a form selected from a food product, a food supplement, a dietary supplement, a meal substitute, a beverage, a beverage supplement, and a pharmaceutical product.

6. Composition for use according to any one of the preceding claims, in the form of a pharmaceutical product, further comprising one or more carriers and/or excipients.

7. Non-therapeutic method for controlling a subject's body weight, said method comprising administering an effective amount of an oral composition comprising a combination of plants, said combination being a combination of *Daucus carota* seed and *Rosa canina* fruit, or extracts thereof obtained by dehydration, cold drying, solid/liquid extraction, grinding or cutting.
